# EUROPEAN PATENT APPLICATION

(11) **EP 4 596 689 A1**
(43) Date of publication of application: **06.08.2025**
(21) Application number: 23872607.9
(22) Date of filing: 29.09.2023
(51) Int. Cl.: C12N 9/50, A23L 5/00, A23L 27/00, A23L 27/20, A61K 38/48, C12N 9/62, C12N 15/57

(54) **ENZYME AGENT AND USE THEREOF**

(30) Priority: 30.09.2022 JP 2022157607
(71) Applicant: Amano Enzyme Inc., Nagoya-shi Aichi 460-8630 (JP); Meijo University, Nagoya-shi, Aichi 468-8502 (JP)
(72) Inventor: SAKAI Kiyota, Kakamigahara-shi, Gifu 509-0109 (JP); SHIMIZU Motoyuki, Nagoya-shi, Aichi 468-8502 (JP); FURUSAWA Terumi, Nagoya-shi, Aichi 468-8502 (JP); NOMURA Ryo, Nagoya-shi, Aichi 468-8502 (JP); KAMEYAMA Ayane, Nagoya-shi, Aichi 468-8502 (JP); KATO Masashi, Nagoya-shi, Aichi 468-8502 (JP)
(74) Representative: Witthoff Jaekel Steinecke Patentanwälte PartG mbB
(86) International application number: PCT/JP2023/035670
(87) International publication number: WO 2024/071388

(57) **Abstract**

It is an object of the present invention to provide an enzyme agent containing a novel aminopeptidase exhibiting substrate specificity that is different from those of existing aminopeptidases. The present invention relates to an enzyme agent containing, as an active ingredient, an aminopeptidase consisting of the amino acid sequence as set forth in SEQ ID No: 1 or SEQ ID No: 8 or an amino acid sequence equivalent thereto. Moreover, the present invention relates to an enzyme agent containing, as an active ingredient, an aminopeptidase, which is derived from *Aspergillus nidulans or Aspergillus oryzae* and has the following physicochemical properties:
(1) optimal temperature: around 50°C;
(2) optimal pH: around pH 7 to 8; and
(3) substrate specificity: having the highest specificity to L-alanine residue or L-lysine residue.

## Description

### Technical Field

The present invention relates to an enzyme agent containing a novel aminopeptidase as an active ingredient, and application thereof.

### Background Art

Amino acids and peptides are widely used in medicines and food products. Amino acids and peptides can be obtained mainly by decomposing proteins. As decomposition methods, an acid hydrolysis method and an enzymatic decomposition method are known, and the decomposition methods greatly influence on the taste. In recent years, needs for taste have become more diverse, and there is a demand for improving the taste of vegetable protein food products due to health consciousness. For example, it is known that the taste components of amino acids, namely, valine, leucine, isoleucine, phenylalanine, tyrosine and tryptophan, are involved in bitter taste (Patent Document 1). Moreover, in addition to glutamic acid, glycine and alanine are also involved in *umami* taste, and it has been reported that arginine is involved in the rich taste of marine products (Non-Patent Document 1).

Aminopeptidase is an enzyme (exopeptidase) that catalyzes the cleavage of amino acid units from the amino terminus (N-terminus) of proteins and peptides. Aminopeptidase is widely distributed throughout animals, plants and microorganisms as a whole, and is present in many intracellular organelles, cytosols, membrane components, and extracellular regions.

As such aminopeptidases, amino peptidases having various substrate specificities have been reported. For example, Patent Document 1 discloses that aminopeptidase derived from *Aspergillus oryzae* can be used to improve breads and has the effect of removing bitter taste. Patent Document 2 discloses leucine aminopeptidase derived from *Aspergillus sojae* that can be used to produce seasonings such as soy sauce. Patent Document 3 discloses glutamic acid aminopeptidase derived from *Aspergillus oryzae* that can improve the *umami* taste of seasonings. Patent Document 4 discloses that glycine aminopeptidase derived from *Actinomucor elegans* has been disclosed, which can be used to produce seasonings with strong taste. Patent Document 5 discloses alanine aminopeptidase derived from Aeromonas sp., which is useful for improving the taste and flavor of edible meats.

### Prior Art Documents

### Patent Documents

Patent Document 1: JP Patent Publication No. 11-509082 A (1999)
Patent Document 2: JP Patent Publication No. 11-346777 A (1999)
Patent Document 3: JP Patent Publication No. 2008-11710 A
Patent Document 4: JP Patent Publication No. 2001-17165 A
Patent Document 5: JP Patent Publication No. 7-289256 A (1995)

### Non-Patent Documents

Non-Patent Document *1:* Seibutsu Kogaku (Bioengineering), Volume 89, 2011, No. 11, PP.679 to 682

### Summary of Invention

### Objects to be Solved by the Invention

As mentioned above, aminopeptidases having various substrate specificities are known. In recent years, however, there has been an increasing need to modify the taste of protein-containing food products, and aminopeptidases exhibiting substrate specificities that are different from those of existing aminopeptidases are in demand. Thus, it is an objective of the present invention to provide an enzyme agent containing a novel aminopeptidase exhibiting substrate specificity that is different from those of existing aminopeptidases.

### Means for Solving the Objects

In order to achieve the above-described object, the present inventors have focused on proteins with unknown functions that are secreted by microorganisms in response to proteins, have prepared, as recombinant proteins, proteins whose functions could not be predicted from their primary sequences, and have then analyzed the functions of such recombinant proteins. As a result of extensive studies, the present inventors have discovered a novel aminopeptidase having substrate specificity that is different from those of existing aminopeptidases, thereby completing the present invention.

Specifically, the present invention has the following configurations.
<1> An enzyme agent containing, as an active ingredient, an aminopeptidase consisting of the amino acid sequence as set forth in SEQ ID No: 1 or an amino acid sequence equivalent thereto.
<2> The enzyme agent according to <1>, in which the aminopeptidase consisting of the equivalent amino acid sequence has an identity of 90% or more to the amino acid sequence as set forth in SEQ ID No: 1 and has an aminopeptidase activity.
<3> The enzyme agent according to <1> or <2>, in which the aminopeptidase is derived from *Aspergillus nidulans.*
<4> An enzyme agent containing, as an active ingredient, an aminopeptidase, which is derived from *Aspergillus nidulans* and has the following physicochemical properties:
   (1) optimal temperature: around 50°C;
   (2) optimal pH: around pH 7 to 8; and
   (3) substrate specificity: having the highest specificity to L-alanine residue.
<5> An enzyme agent containing, as an active ingredient, an aminopeptidase consisting of the amino acid sequence as set forth in SEQ ID No: 8 or an amino acid sequence equivalent thereto.
<6> The enzyme agent according to <5>, in which the aminopeptidase consisting of the equivalent amino acid sequence has an identity of 90% or more to the amino acid sequence as set forth in SEQ ID No: 8 and has an aminopeptidase activity.
<7> The enzyme agent according to <5> or <6>, in which the aminopeptidase is derived from *Aspergillus oryzae.*
<8> The enzyme agent according to any one of <5> to <7>, which has the highest specificity to L-lysine residue.
<9> The enzyme agent according to any one of <1> to <8>, which is for use in modification of the taste of a food product.
<10> A method for producing a food product or a food product material, which includes allowing the enzyme agent according to any one of <1> to <8> to act on a protein-containing raw material.
<11> A method for producing a seasoning, which includes allowing the enzyme agent according to any one of <1> to <8> to act on at least one type selected from proteins and peptides.
<12> A method for modifying the taste of a food product, which includes allowing the enzyme agent according to any one of <1> to <8> to act on a protein-containing raw material.
<13> A food product, containing the enzyme agent according to any one of <1> to <8> and at least one type selected from proteins and peptides.
<14> A seasoning, containing the enzyme agent according to any one of <1> to <8> and at least one type selected from proteins and peptides.

### Advantageous Effects of Invention

According to the present invention, there can be provided an enzyme agent containing a novel aminopeptidase having substrate specificity that is different from those of existing aminopeptidases.

### Brief Description of Drawings

[Fig. 1] Fig. 1 is a graph showing the optimal temperature of an aminopeptidase consisting of the amino acid sequence as set forth in SEQ ID No: 1.
[Fig. 2] Fig. 2 is a graph showing the optimal pH of an aminopeptidase consisting of the amino acid sequence as set forth in SEQ ID No: 1.
[Fig. 3] Fig. 3 is a graph showing the enzyme activity of an aminopeptidase consisting of the amino acid sequence as set forth in SEQ ID No: 1.

### Embodiment of Carrying out the Invention

Hereafter, the present invention will be described in detail. The configuration requirements described below may be described based on representative embodiments or specific examples, but the present invention is not limited to such embodiments. In the present description, a numerical value range expressed using the preposition "to" means a range that includes the numerical values described before and after the preposition "to" as a lower limit value and a upper limit value, respectively.

In the present description, the 20 types of amino acid residues in the amino acid sequences may be expressed by one-letter abbreviations in some cases. In that case, glycine (Gly) is G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, threonine (Thr) is T, cysteine (Cys) is C, methionine (Met) is M, Aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P. Moreover, in the present description, in the amino acid sequences displayed, the N-terminus is at the left end and the C-terminus is at the right end.

### 1. Aminopeptidase-containing enzyme agent

A first aspect of the present invention relates to an enzyme agent containing, as an active ingredient, an aminopeptidase consisting of the amino acid sequence as set forth in SEQ ID No: 1 or an amino acid sequence equivalent thereto. In addition, a second aspect of the present invention relates to an enzyme agent containing, as an active ingredient, an aminopeptidase consisting of the amino acid sequence as set forth in SEQ ID No: 8 or an amino acid sequence equivalent thereto. The enzyme agent of the present invention (hereinafter also referred to as "the present enzyme agent") contains an aminopeptidase (hereinafter also referred to as "the present enzyme") as an active ingredient.

The aminopeptidase serving as an active ingredient, namely, the present enzyme consists of the amino acid sequence as set forth in SEQ ID No: 1 or an amino acid sequence equivalent to the amino acid sequence as set forth in SEQ ID No: 1, or the amino acid sequence as set forth in SEQ ID No: 8 or an amino acid sequence equivalent to the amino acid sequence as set forth in SEQ ID No: 8. Herein, the "equivalent amino acid sequence" means an amino acid sequence that is partially different from the reference amino acid sequence (i.e. the amino acid sequence as set forth in SEQ ID No: 1 or the amino acid sequence as set forth in SEQ ID No: 8) but such difference does not substantially influence on the function of the protein (which is herein an aminopeptidase activity). In addition, in the present invention, the "equivalent amino acid sequence" means an amino acid sequence having an equivalent substrate specificity.

Aminopeptidase is an enzyme that cleaves a peptide bond from the amino terminus (N-terminus) of a protein or a peptide to release an amino acid. In particular, in the first aspect, the present enzyme has the activity of releasing alanine from the amino terminus of a protein or a peptide (alanine aminopeptidase activity). The degree of the alanine aminopeptidase activity possessed by an enzyme consisting of an amino acid sequence that is equivalent to the amino acid sequence as set forth in SEQ ID No: 1 is not particularly limited, as long as the enzyme is capable of exerting the function of alanine aminopeptidase. However, it is preferable that the degree of the alanine aminopeptidase activity possessed by the enzyme consisting of an amino acid sequence that is equivalent to the amino acid sequence as set forth in SEQ ID No: 1 is the same as or higher than that of an enzyme consisting of a reference amino acid sequence (an enzyme having the amino acid sequence as set forth in SEQ ID No: 1). The same degree of alanine aminopeptidase activity means that the present enzyme has an alanine-releasing ability that is ±20%, or preferably ±10% of the alanine-releasing ability of alanine aminopeptidase having the reference amino acid sequence.

Moreover, in the second aspect, the present enzyme has the activity of releasing lysine from the amino terminus of a protein or a peptide (lysine aminopeptidase activity). The degree of the lysine aminopeptidase activity possessed by an enzyme consisting of an amino acid sequence that is equivalent to the amino acid sequence as set forth in SEQ ID No: 8 is not particularly limited, as long as the enzyme is capable of exerting the function of lysine aminopeptidase. However, it is preferable that the degree of the lysine aminopeptidase activity possessed by the enzyme consisting of an amino acid sequence that is equivalent to the amino acid sequence as set forth in SEQ ID No:8 is the same as or higher than that of an enzyme consisting of a reference amino acid sequence (an enzyme having the amino acid sequence as set forth in SEQ ID No: 8). The same degree of lysine aminopeptidase activity means that the present enzyme has an lysine-releasing ability that is ±20%, or preferably ±10% of the lysine-releasing ability of lysine aminopeptidase having the reference amino acid sequence.

The present enzyme agent contains a novel aminopeptidase consisting of the amino acid sequence as set forth in SEQ ID No: 1 or an amino acid sequence equivalent thereto, or a novel aminopeptidase consisting of the amino acid sequence as set forth in SEQ ID No: 8 or an amino acid sequence equivalent thereto, and thus, the present invention provides a novel enzyme agent. Furthermore, the present enzyme is a novel aminopeptidase having substrate specificity that is different from those of existing aminopeptidases. Since the substrate specificity of the present enzyme is relatively wide, it is expected that, for example, the present enzyme provides the effect of modifying the taste of food products that is different from those of existing aminopeptidases.

In the first aspect, the present enzyme is preferably derived from *Aspergillus nidulans.* That is, the amino acid sequence as set forth in SEQ ID No: 1 is preferably the amino acid sequence (mature body) of the aminopeptidase derived from *Aspergillus nidulans.* It is to be noted that the full-length amino acid sequence of the aminopeptidase derived from *Aspergillus nidulans* having a signal peptide is shown in SEQ ID No: 2.

In the second aspect, the present enzyme is preferably derived from *Aspergillus oryzae.* That is, the amino acid sequence as set forth in SEQ ID No: 8 is preferably the amino acid sequence (mature body) of the aminopeptidase derived from *Aspergillus oryzae.* It is to be noted that the full-length amino acid sequence of the aminopeptidase derived from *Aspergillus oryzae* having a signal peptide is shown in SEQ ID No: 9.

The amino acid sequence of an aminopeptidase consisting of an equivalent amino acid sequence is partially different from the reference amino acid sequence (the amino acid sequence as set forth in SEQ ID No: 1 or the amino acid sequence as set forth in SEQ ID No: 8). Herein, when the amino acid sequence "is partially different from the reference amino acid sequence," for example, a deletion or a substitution of one or multiple amino acids in the amino acids constituting the amino acid sequence, or an addition or an insertion of one or multiple amino acids into the amino acid sequence, or any given combination thereof, is generated. Such a partial difference in the amino acid sequence is acceptable as long as the aminopeptidase activity is retained, and it is appropriate even if the activity may be slightly fluctuated. As far as this condition is satisfied, the position of the amino acid sequence, in which the difference is found, is not particularly limited. In addition, such difference may be generated in multiple sites (places) on the amino acid sequence.

The number of different sites on the amino acid sequence is preferably 30% or less, more preferably 20% or less, even more preferably 15% or less, further preferably 10% or less, even further preferably 5% or less, still further preferably 3% or less, still further preferably 2% or less, still further preferably 1% or less, still further preferably 0.7% or less, particularly preferably 0.5% or less, even particularly preferably 0.3% or less, and most preferably 0.1% or less, with respect to the entire amino acids that constitute the amino acid sequence. Accordingly, the aminopeptidase consisting of the equivalent protein has an identity of preferably 70% or more, more preferably 80% or more, even more preferably 85% or more, further preferably 90% or more, even further preferably 95% or more, still further preferably 97% or more, still further preferably 98% or more, still further preferably 99% or more, particularly preferably 99.3% or more, even particularly preferably 99.5% or more, further particularly preferably 99.7% or more, and most preferably 99.9% or more, to the reference amino acid sequence (the amino acid sequence as set forth in SEQ ID No: 1 or the amino acid sequence as set forth in SEQ ID No: 8).

A typical example of the case of "being partially different from the reference amino acid sequence" is that a mutation (a change) is generated in the amino acid sequence by a deletion or a substitution of 1 to 40 (preferably 1 to 30, more preferably 1 to 10, even more preferably 1 to 7, further preferably 1 to 5, and still further preferably 1 to 3) amino acids in the amino acids constituting the amino acid sequence, or an addition or an insertion of 1 to 40 (preferably 1 to 30, more preferably 1 to 10, even more preferably1 to 7, further preferably 1 to 5, and still further preferably 1 to 3) amino acids into an amino acid sequence, or any given combination thereof.

In the case of "being partially different from the reference amino acid sequence," it is preferable that a conservative amino acid substitution occurs in amino acid residues that are not essential for aminopeptidase activity, and thereby, an equivalent amino acid sequence is easily obtained. The term "conservative amino acid substitution" is used herein to mean that a certain amino acid residue is substituted with an amino acid residue having a side chain with similar properties. Amino acid residues are classified into several families, depending on the side chain thereof; namely, basic side chains (for example, lysine, arginine, and histidine), acidic side chains (for example, aspartic acid and glutamic acid), uncharged polar side chains (for example, glycine, asparagine, glutamine, serine, threonine, tyrosine, and cysteine), non-polar side chains (for example, alanine, valine, leucine, isoleucine, proline, phenylalanine, methionine, and tryptophan), β-branched side chains (for example, threonine, valine, and isoleucine), aromatic side chains (for example, tyrosine, phenylalanine, tryptophan, and histidine), etc. In the conservative amino acid substitution, preferably, a substitution occurs between amino acid residues in an identical family.

The identity (%) of two amino acid sequences can be determined, for example, by the following procedures. First, two sequences are aligned such that an optimal comparison can be made (for example, a gap may be introduced into a first sequence, so that the alignment with a second sequence may be optimized). When a molecule (amino acid residue) in a specific position of the first sequence is identical to a molecule (amino acid residue) in a corresponding position in the second sequence, it can be said that the molecules (amino acid residues) at the positions are identical to each other. The identity of the two sequences is calculated from the number of identical positions common in the two sequences (identity (%) = the number of identical positions / total number of positions x 100). Besides, in this case, it is preferable that the number and size of gaps used for optimization of the alignment are also taken into consideration.

Comparison of two sequences and determination of identity can be realized using mathematical algorithms. A specific example of the mathematical algorithm that can be utilized in comparison of sequences may be an algorithm that is described in Karlin and Altschul (1990) Proc. Natl. Acad. Sci. USA 87: 2264-68 and is modified in Karlin and Altschul (1993) Proc. Natl. Acad. Sci. USA 90: 5873-77, but the example of the mathematical algorithm is not limited thereto. The identity of amino acid sequences can be obtained, for example, by blastp (protein-protein BLAST) of National Center for Biotechnology Information (NCBI). As parameters, default parameters may be used. For example, using BLOSUM62 matrix, Gap Costs may be set to be Existence: 11 and Extension: 1.

The aminopeptidase that is an active ingredient of the present enzyme agent, namely, the present enzyme may be a part of a larger protein (for example, a fusion protein). Examples of a sequence to be added to such a fusion protein may include sequences that are useful for purification, such as multiple histidine residues, and additional sequences that ensure stability during recombinant production.

The present enzyme can be obtained by culturing microorganisms that produce the present aminopeptidase (i.e. an aminopeptidase-producing strain). In the first aspect, the present enzyme can be obtained by culturing *Aspergillus nidulans,* specifically, *Aspergillus nidulans* A26 strain (obtained from Fungal Genetic Stock Center (Kansas State University)). Moreover, in the second aspect, the present enzyme can be obtained by culturing *Aspergillus oryzae.* The aminopeptidase-producing strain may be either a wild-type strain or a mutant strain (such a mutant strain is obtained, for example, by ultraviolet irradiation).

The present enzyme can be prepared from a culture solution and/or a cell mass of microorganisms that produce the present enzyme. The culture conditions or the culture methods are not particularly limited, as long as the present enzyme can be produced thereby. That is to say, the methods or the culture conditions that are adapted to the culture of the used microorganisms can be determined, as appropriate, under conditions in which the present enzyme is produced. Regarding the culture method, either liquid culture or solid culture may be applied, and liquid culture is preferably utilized. Hereafter, taking the liquid culture as an example, the culture conditions will be described.

The medium is not particularly limited, as long as it is a medium in which the used microorganisms can grow. Examples of the medium that can be used herein may include those, to which the following substances are added: carbon sources, such as glucose, sucrose, gentiobiose, soluble starch, glycerin, dextrin, molasses, and organic acids; nitrogen sources, such as ammonium sulfate, ammonium carbonate, ammonium phosphate, ammonium acetate, or such as gelatin, peptone, yeast extract, corn steep liquor, casein hydrolysate, bran, and meat extract; and further, inorganic salts, such as potassium salts, magnesium salts, sodium salts, phosphates, manganese salts, iron salts and zinc salts. In order to promote the growth of the used microorganisms, vitamins, amino acids and the like may be added to the medium. The pH of the medium is preferably, for example, approximately pH 3 to 8, and is more preferably approximately pH 4 to 7. The culture temperature is, in general, preferably about 20°C to 40°C, and is more preferably about 25°C to 35°C. The microorganisms are cultured for 1 to 20 days, and preferably for 3 to 10 days, under aerobic conditions. As a culture method applied herein, for example, a shaking culture method, or an aerobic deep culture method using a jar fermenter, can be utilized.

After completion of the culture performed under the above-described conditions, the enzyme of interest is recovered from the culture solution or the culture mass. When the enzyme is recovered from the culture solution, for example, the culture supernatant is filtered, centrifuged, etc. to remove insoluble matters, and thereafter, the resultant is separated and purified by appropriately combining concentration using an ultrafiltration membrane, salting-out such as ammonium sulfate precipitation, dialysis, and various types of chromatography using ion exchange resin, etc., thereby obtaining the present enzyme. On the other hand, when the enzyme is recovered from the cell mass, for example, the cell mass is crushed by a pressure treatment, an ultrasonic treatment, etc., and is then separated and purified in the same manner as that described above, so as to obtain the present enzyme. Besides, the cell mass may be previously recovered from the culture solution by filtration, a centrifugation treatment, etc., and thereafter, the above-described series of steps (crushing, separation, and purification of the cell mass) may be carried out.

Also, the present enzyme can be easily prepared by a genetic engineering technique. For example, the present enzyme can be prepared by transforming suitable host cells (for example, *Escherichia coli)* with DNA encoding the present enzyme, and then recovering the protein expressed in the transformant. The recovered protein is appropriately purified, depending on the purpose. Thus, the enzyme of interest can be obtained as a recombinant protein, and according to this method, various modifications can be carried out. For example, a DNA encoding the present enzyme and another appropriate DNA are inserted into an identical vector, and a recombinant protein is produced using the vector, so that the present enzyme consisting of a recombinant protein, in which any given peptides or proteins are ligated to each other, can be obtained. Also, modification can be carried out on the present enzyme, so that a sugar chain and/or a lipid may be added, or that the processing of the N-terminus or the C-terminus may be generated. By the above-described method, simplification of the extraction and purification of a recombinant protein, addition of biological functions, etc. can be carried out.

Generally, the expression of a gene is carried out utilizing an appropriate host-vector system as described above, and a gene expression product (the present enzyme) is recovered. However, a cell-free synthesis system (a cell-free transcription system or a cell-free transcription/translation system) may also be utilized. Herein, the term "cell-free synthesis system (a cell-free transcription system or a cell-free transcription/translation system)" means that, not using living cells, but using a ribosome, a transcription/translation factor or the like derived from living cells (or obtained by a genetic engineering technique), from a nucleic acid (DNA or mRNA) used as a template, mRNA or protein encoded thereby is synthesized *in vitro.* In general, in the cell-free synthesis system, a cell extract obtained by purifying, as necessary, a cell-disintegrated solution is used. The cell extract generally contains a ribosome, various types of factors such as an initiation factor, and various types of enzymes such as tRNA, which are necessary for protein synthesis. When a protein is synthesized, various types of amino acids, energy sources such as ATP or GTP, and other substances necessary for protein synthesis, such as creatine phosphate, are added to the aforementioned cell extract. Upon the synthesis of a protein, a ribosome, various types of factors, and/or various types of enzymes, and the like, which are prepared separately, may also be replenished to the aforementioned cell extract, as necessary.

The development of a transcription/translation system, in which various molecules (factors) necessary for protein synthesis have been reconstructed, has also been reported (Shimizu, Y. et al.: Nature Biotech., 19, 751-755, 2001). In this synthesis system, genes of 31 types of factors composed of: 3 types of initiation factors, 3 types of elongation factors, 4 types of factors associated with termination, 20 types of aminoacyl tRNA synthesis enzymes that allow each amino acid to bind to tRNA, and a methionyl tRNA formyl transfer enzyme, which constitute a protein synthesis system of bacteria, are amplified from an *Escherichia coli* genome, and then, using these amplified products, a protein synthesis system is reconstructed *in vitro.* In the present invention, such a reconstructed synthesis system may be utilized.

The "cell-free transcription system or cell-free transcription/translation system " is exchangeably used with the term "cell-free protein synthesis system," *"in vitro* translation system" or *"in vitro* transcription/translation system." In the *in vitro* translation system, RNA is used as a template to synthesize a protein. As such template RNA, total RNA, mRNA, an *in vitro* transcriptional product or the like is used. On the other hand, in the *in vitro* transcription/translation system, DNA is used as a template. The template DNA preferably contains a ribosome-binding region, and also preferably contains an appropriate terminator sequence. In addition, in the *in vitro* transcription/translation system, in order to promote continuous progression of the transcription reaction and the translation reaction, conditions, in which factors necessary for each reaction are added, are established.

The purified enzyme that is obtained as described above is pulverized, for example, by freeze drying, vacuum drying, or spray drying, so that the enzyme can be provided in the form of powders.

The purified enzyme may be dissolved in water. Preferably, the purified enzyme is dissolved in a buffer. Examples of the buffer may include an acetate buffer, a phosphate buffer, a triethanolamine buffer, a Tris-HCl buffer, and a GOOD buffer. The GOOD buffer may be PIPES, MES, or MOPS.

The purification degree of the enzyme is not particularly limited, and for example, the enzyme can be purified, so that the aminopeptidase activity becomes 1 to 2000 (U/g), preferably 10 to 1500 (U/g), and more preferably 100 to 1000 (U/g). In addition, the final form of the enzyme may be either a liquid or a solid (including powders).

A third aspect of the present invention relates to an enzyme agent containing, as an active ingredient, an aminopeptidase, which is derived from *Aspergillus nidulans* and has the following physicochemical properties:
(1) optimal temperature: around 50°C;
(2) optimal pH: around pH 7 to 8; and
(3) substrate specificity: having the highest specificity to L-alanine residue.

As a result of the studies conducted by the present inventors, the properties of an *Aspergillus nidulans-derived* aminopeptidase consisting of the amino acid sequence as set forth in SEQ ID No: 1 have been determined as follows. Accordingly, in the third aspect of the present invention, the present enzyme can also be specified by the above-described chemical properties of the enzyme. It is to be noted that the details of the measuring conditions, measuring procedures, etc. of aminopeptidase activity upon evaluation of individual chemical properties of the enzyme are as described in the after-mentioned Examples.

### < Action >

The present enzyme in the third aspect is an aminopeptidase, and the aminopeptidase acts on a protein or a peptide to decompose the peptide bond at the amino terminus. In particular, it is preferable that the present enzyme has an activity of releasing alanine from the amino terminus of a protein or a peptide (alanine aminopeptidase activity).

### < Optimal temperature >

The optimal temperature of the present enzyme in the third aspect is around 50°C. More specifically, the optimal temperature of the present enzyme is preferably 40°C to 60°C, and more preferably 45°C to 55°C.

< Optimal pH >

The optimal pH of the present enzyme in the third aspect is pH 7 to 8. For example, in the pH range of pH 3 to 7, the optimal pH is determined based on the results measured in a McIlvaine buffer solution; in the pH range of pH 6 to 7, the optimal pH is determined based on the results measured in a Tris-HCl buffer solution; and in the pH range of pH 8 to 11, the optimal pH is determined based on the results measured in a CAPS buffer.

### < Substrate specificity >

The present enzyme in the third aspect preferably has the activity of releasing alanine from the amino terminus of a protein or a peptide (alanine aminopeptidase activity). For example, when the following various types of amino acid-p-nitroanilides (Ala-pNA, Gly-pNA, Lys-pNA, Val-pNA, Arg-pNA, Phe-pNA, Pro-pNA, Ile-pNA, Glu-pNA, Leu-pNA, and Asp-pNA) are used as substrates, the present enzyme exhibits the highest activity to alanine-p-nitroanilide (Ala-pNA). Among them, the present enzyme preferably has the highest specificity to L-alanine residues.

The present enzyme in the third aspect exhibits an activity to hydrophobic amino acids (alanine, glycine, valine, and phenylalanine) and some basic amino acids (lysine and arginine). For example, when the activity value of alanine-p-nitroanilide (Ala-pNA) is set to be 100%, the activity value of glycine-p-nitroanilide (Gly-pNA) may be 40% or more, the activity value of lysine-p-nitroanilide (Lys-pNA) may be 30% or more, the activity value of valine-p-nitroanilide (Val-pNA) may be 20% or more, the activity value of arginine-p-nitroanilide (Arg-pNA) may be 20% or more, and the activity value of phenylalanine-p-nitroanilide (Phe-pNA) may be 10% or more.

### < Molecular weight >

The molecular weight of the present enzyme in the third aspect is approximately 47 kDa, based on the theoretical value from the amino acid sequence.

The content of the active ingredient (the present enzyme) in the present enzyme agent in the third aspect is not particularly limited, and for example, it is preferable to set or adjust the content of \the active ingredient (the present enzyme), so that the alanine aminopeptidase activity per gram of the present enzyme agent becomes 0.001 U to 10,000 U, 0.005 U to 5,000 U, 0.01 U to 2,000 U, 0.1 U to 1,000 U, and preferably 1 U to 500 U.

In a fourth aspect, the present enzyme agent preferably has the highest specificity to L-lysine residues. As a result of the studies conducted by the present inventors, the properties of an *Aspergillus* oryzae-derived aminopeptidase consisting of the amino acid sequence as set forth in SEQ ID No: 8 have been determined as follows. It is to be noted that the details of the measuring conditions, measuring procedures, etc. of aminopeptidase activity upon evaluation of individual chemical properties of the enzyme are as described in the after-mentioned Examples.

### < Action >

The present enzyme in the fourth aspect is an aminopeptidase, and the aminopeptidase acts on a protein or a peptide to decompose the peptide bond at the amino terminus. In particular, it is preferable that the present enzyme has an activity of releasing lysine from the amino terminus of a protein or a peptide (lysine aminopeptidase activity).

### < Substrate specificity >

The present enzyme in the fourth aspect preferably has the activity of releasing lysine from the amino terminus of a protein or a peptide (lysine aminopeptidase activity). For example, when the following various types of amino acid-p-nitroanilides (Ala-pNA, Gly-pNA, Lys-pNA, Val-pNA, Arg-pNA, Phe-pNA, Pro-pNA, Ile-pNA, Glu-pNA, Leu-pNA, and Asp-pNA) are used as substrates, the present enzyme exhibits the highest activity to lysine-p-nitroanilide (Lys-pNA). Among them, the present enzyme preferably has the highest specificity to L-lysine residues.

The present enzyme in the fourth aspect exhibits an activity to hydrophobic amino acids (alanine, glycine, valine, and phenylalanine) and some basic amino acids (lysine and arginine). For example, when the activity value of lysine-p-nitroanilide (Lys-pNA) is set to be 100%, the activity value of glycine-p-nitroanilide (Gly-pNA) may be 40% or more, the activity value of alanine-p-nitroanilide (Ala-pNA) may be 30% or more, the activity value of valine-p-nitroanilide (Val-pNA) may be 15% or more, the activity value of arginine-p-nitroanilide (Arg-pNA) may be 10% or more, and the activity value of leucine-p-nitroanilide (Leu-pNA) may be 10% or more.

### < Molecular weight >

The molecular weight of the present enzyme in the fourth aspect is approximately 47 kDa, based on the theoretical value from the amino acid sequence.

The content of the active ingredient (the present enzyme) in the present enzyme agent in the fourth aspect is not particularly limited, and it is preferable to set or adjust the content of the active ingredient (the present enzyme), so that the lysine aminopeptidase activity per gram of the present enzyme agent becomes, for example, 0.001 U to 10,000 U, 0.005 U to 5,000 U, 0.01 U to 2,000 U, 0.1 U to 1,000 U, and preferably 1 U to 500 U.

### < Optional components of aminopeptidase-containing enzyme agent >

The enzyme agent of the present invention is usually provided in the form of a solid (for example, an immobilized enzyme formed by immobilizing the present enzyme on a material capable of immobilizing the enzyme on the surface or inside thereof, such as a granule, a powder, a silica, or a porous polymer) or a liquid. The present enzyme agent may contain an excipient, a buffer agent, a suspending agent, a stabilizer, a preservative, an antiseptic, a normal saline, etc., as well as the active ingredient (the present enzyme). As such an excipient, lactose, sorbitol, D-mannitol, maltodextrin, saccharose, etc. can be used. As such a buffer agent, phosphate, citrate, acetate, etc. can be used. As such a stabilizer, propylene glycol, ascorbic acid, etc. can be used. As such a preservative, phenol, benzalkonium chloride, benzyl alcohol, chlorobutanol, methylparaben, etc. can be used. As such an antiseptic, benzalkonium chloride, paraoxybenzoic acid, chlorobutanol, etc. can be used.

The enzyme agent of the present invention may contain metal ions in addition to the active ingredient (the present enzyme). Examples of the metal ions may include Cu²⁺, Zn²⁺, Mn²⁺, Mg²⁺, Ca²⁺, Co²⁺, and Fe²⁺. These metal ions may be added as water-soluble metal salts such as a chloride, a sulfate, an acetate, and a nitrate. Among them, the metal ion is preferably at least one type selected from the group consisting of Cu²⁺, Mn²⁺, and Mg²⁺. By allowing such metal ions to coexist, substrate specificity can be more effectively increased, and enzyme activity can be enhanced.

The metal ion concentration in the reaction solution during the enzyme reaction is preferably 0.001 mM or more, and more preferably 0.01 mM or more. In addition, the metal ion concentration in the reaction solution during the enzyme reaction is preferably 100 mM or less, and more preferably 10 mM or less.

### 2. Gene

A fifth aspect of the present invention relates to cDNA consisting of the nucleotide sequence as set forth in SEQ ID No: 3, cDNA consisting of the nucleotide sequence as set forth in SEQ ID No: 4, and genomic DNA consisting of the nucleotide sequence as set forth in SEQ ID No: 5, which are genes encoding an aminopeptidase consisting of the amino acid sequence as set forth in SEQ ID No: 1 or an amino acid sequence equivalent thereto. In addition, a sixth aspect of the present invention relates to cDNA consisting of the nucleotide sequence as set forth in SEQ ID No: 10, cDNA consisting of the nucleotide sequence as set forth in SEQ ID No: 11, and genomic DNA consisting of the nucleotide sequence as set forth in SEQ ID No: 12, which are genes encoding an aminopeptidase consisting of the amino acid sequence as set forth in SEQ ID No: 8 or an amino acid sequence equivalent thereto. Also, the present enzyme may be produced by obtaining a gene encoding the present enzyme and then allowing the obtained gene to express.

In the fifth aspect, the gene encoding the present enzyme includes DNA encoding the amino acid sequence as set forth in SEQ ID No: 1. Specific examples of the present aspect may include the cDNA consisting of the nucleotide sequence as set forth in SEQ ID No: 3, the cDNA consisting of the nucleotide sequence as set forth in SEQ ID No: 4, and the genomic DNA consisting of the nucleotide sequence as set forth in SEQ ID No: 5. The DNA of SEQ ID No: 3 encodes only the amino acid sequence (SEQ ID No: 1) of a mature body, the DNA of SEQ ID No: 4 encodes a signal peptide in addition to the mature body (the amino acid sequence as set forth in SEQ ID No: 1). The DNA of SEQ ID No: 5 contains an intron in addition to the sequences encoding the mature body and the signal peptide.

Moreover, in the sixth aspect, the gene encoding the present enzyme includes DNA encoding the amino acid sequence as set forth in SEQ ID No: 8. Specific examples of the present aspect may include the cDNA consisting of the nucleotide sequence as set forth in SEQ ID No: 10, the cDNA consisting of the nucleotide sequence as set forth in SEQ ID No: 11, and the genomic DNA consisting of the nucleotide sequence as set forth in SEQ ID No: 12. The DNA of SEQ ID No: 10 encodes only the amino acid sequence (SEQ ID No: 8) of a mature body, the DNA of SEQ ID No: 11 encodes a signal peptide in addition to the mature body (the amino acid sequence as set forth in SEQ ID No: 8). The DNA of SEQ ID No: 12 contains an intron in addition to the sequences encoding the mature body and the signal peptide.

The gene encoding the present enzyme is typically utilized in preparation of the present enzyme. According to a genetic engineering preparation method using the gene encoding the present enzyme, it is possible to obtain the present enzyme in a more homogeneous state. In addition, it is said that this method is a method preferably used in the case of preparing a large amount of the present enzyme. Besides, the intended use of the gene encoding the present enzyme is not limited to the preparation of the present enzyme. For example, the present nucleic acid can also be used as an experimental tool for elucidating the action mechanism of the present enzyme, or as a tool for designing or preparing a mutant (a modified body) of the present enzyme.

In the present description, the phrase "the gene encoding the present enzyme" means a nucleic acid that provides the present enzyme, when the nucleic acid is allowed to express. Thus, the gene encoding the present enzyme includes not only a nucleic acid having a nucleotide sequence corresponding to the amino acid sequence of the present enzyme, but also a nucleic acid formed by adding a sequence that does not encode the amino acid sequence to the aforementioned nucleic acid. Moreover, codon degeneracy is also taken into consideration.

The nucleic acid can be prepared in an isolated state according to a standard genetic engineering technique, a molecular biological technique, a biochemical technique, chemical synthesis, a PCR method (for example, overlap PCR), or a combination thereof, with reference to the present description or the sequence information disclosed in the sequence listing attached herewith.

According to the fifth aspect or the sixth aspect of the present invention, provided is a nucleic acid, in which when the nucleic acid is compared with the nucleotide sequence of the gene encoding the present enzyme, the function of a protein encoded thereby is equivalent, but the nucleotide sequence thereof is partially different from the nucleotide sequence of the gene encoding the present enzyme (which is hereinafter also referred to as an "equivalent nucleic acid," and the nucleotide sequence of the equivalent nucleic acid is also referred to as an "equivalent nucleotide sequence"). An example of such an equivalent nucleic acid may be DNA that consists of a nucleotide sequence containing a substitution, deletion, insertion, addition or inversion of one or multiple nucleotides, based on the nucleotide sequence of the nucleic acid encoding the enzyme of the present invention, and encodes a protein having enzyme activity characteristic of the present enzyme (i.e. aminopeptidase activity). Substitution, deletion, etc. of nucleotides may occur in multiple sites. The term "multiple" is used herein to mean, for example, 2 to 40 nucleotides, preferably 2 to 20 nucleotides, and more preferably 2 to 10 nucleotides, although the number of nucleotides is different depending on the positions or types of amino acid residues in the three-dimensional structure of a protein encoded by the nucleic acid. The equivalent nucleic acid has an identity of, for example, preferably 70% or more, more preferably 80% or more, even more preferably 90% or more, further preferably 92% or more, still further preferably 94% or more, still further preferably 96% or more, still further preferably 98% or more, and particularly preferably 99% or more, with respect to the nucleotide sequence serving as a reference (SEQ ID No: 3 or SEQ ID No: 4, or SEQ ID No: 10 or SEQ ID No: 11).

Such an equivalent nucleic acid as described above can be obtained by, for example, a restriction enzyme treatment, a treatment with exonuclease, DNA ligase, etc., introduction of a mutation according to a site-directed mutagenesis (Molecular Cloning, Third Edition, Chapter 13, Cold Spring Harbor Laboratory Press, New York) or a random mutation introduction method (Molecular Cloning, Third Edition, Chapter 13, Cold Spring Harbor Laboratory Press, New York), etc.. The equivalent nucleic acid can also be obtained by other methods such as ultraviolet irradiation.

When the present enzyme is prepared, there may be used a nucleic acid having a nucleotide sequence complementary to the nucleotide sequence of the gene that encodes the present enzyme. The fifth aspect or the sixth aspect of the present invention provides a nucleic acid having a nucleotide sequence that is, at least, 70%, 80%, 90%, 92%, 94%, 96%, 98% or 99% or more identical to the nucleotide sequence of the gene that encodes the present enzyme, or to a nucleotide sequence complementary to the nucleotide sequence of the gene that encodes the present enzyme.

When the present enzyme is prepared, there may be used a nucleic acid having a nucleotide sequence that hybridizes under stringent conditions to a nucleotide sequence that is complementary to the nucleotide sequence of the gene that encodes the present enzyme or a nucleotide sequence equivalent thereto. The term "stringent conditions" is used herein to mean conditions under which, what is called, a specific hybrid is formed, but a non-specific hybrid is not formed. Such stringent conditions are known to those skilled in the art, and can be determined, for example, with reference to Molecular Cloning (Third Edition, Cold Spring Harbor Laboratory Press, New York) or Current protocols in molecular biology (edited by Frederick M. Ausubel et al., 1987). The stringent conditions may be, for example, conditions in which incubation is performed at about 50°C, using a hybridization solution (50% formamide, 10 x SSC (0.15 M NaCl, 15 mM sodium citrate, pH 7.0), 5 x Denhardt solution, 1% SDS, 10% dextran sulfate, 10 µg/ml denatured salmon sperm DNA, and 50 mM phosphate buffer (pH 7.5)), and thereafter, washing is performed at about 65°C, using 0.1 x SSC and 0.1% SDS. More preferred stringent conditions may be, for example, conditions of using, as a hybridization solution, 50% formamide and 5 x SSC (0.15 M NaCl, 15 mM sodium citrate, pH 7.0), 1 x Denhardt solution, 1% SDS, 10% dextran sulfate, 10 µg/ml denatured salmon sperm DNA, 50 mM phosphate buffer (pH 7.5)).

When the present enzyme is prepared, there may be used a nucleic acid (a nucleic acid fragment) having a part of the nucleotide sequence of the gene that encodes the present enzyme or a nucleotide sequence complementary thereto. Such a nucleic acid fragment can be used to, for example, detect, identify, and/or amplify a nucleic acid having the nucleotide sequence of the gene that encodes the present enzyme, etc. For example, the nucleic acid fragment is designed to, at least, contain a portion that hybridizes to a continuous nucleotide portion (for example, a length consisting of about 10 to about 100 nucleotides, preferably about 20 to about 100 nucleotides, and more preferably about 30 to about 100 nucleotides) in the nucleotide sequence of the gene that encodes the present enzyme. When the nucleic acid fragment is utilized as a probe, it can be labeled. For labeling, for example, a fluorescent substance, an enzyme, or a radioisotope can be used.

When the present enzyme is prepared, there may be used recombinant DNA containing the above-described gene (i.e. the gene encoding the present enzyme). The recombinant DNA is provided in the form of, for example, a vector. The term "vector" is used in the present description to mean a nucleic acid molecule capable of transporting a nucleic acid inserted in the nucleic acid molecule into a target such as a cell.

Depending on intended use purpose (cloning or the expression of a protein), or taking into consideration of the type of a host cell, a suitable vector is selected. Examples of the vector having *Escherichia coli* as a host may include M13 phage or a modified body thereof, λ phage or a modified body thereof, and pBR322 or a modified body thereof (pB325, pAT153, pUC8, etc.). Examples of the vector having yeast as a host may include pYepSec1, pMFa, pYES2, and pPICZ. Examples of the vector having an insect cell as a host may include pAc and pVL. Examples of the vector having a mammalian cell as a host may include pCDM8 and pMT2PC.

The vector is preferably an expression vector. The "expression vector" means a vector that is capable of introducing a nucleic acid inserted in the vector into a cell of interest (a host cell) and that is also capable of allowing the nucleic acid to express in the host cell. The expression vector usually contains a promoter sequence necessary for the expression of the inserted nucleic acid, an enhancer sequence for promoting the expression, and the like. An expression vector containing a selective marker can also be used. In the case of using such an expression vector, the presence or absence of the introduction of the expression vector (and the degree thereof) can be confirmed by utilizing the selective marker.

Insertion of the nucleic acid into the vector, insertion of a selective marker (if necessary), insertion of a promoter (if necessary), and the like can be carried out by using a standard recombinant DNA technique (for example, a publicly known method using restriction enzyme and DNA ligase, which can refer to Molecular Cloning, Third Edition, 1.84, Cold Spring Harbor Laboratory Press, New York).

In terms of the ease of handling, microorganisms such as *Escherichia coli, Bacillus subtilis,* and yeasts *(Saccharomyces cerevisiae* and *Pichia pastoris)* are preferably used as host cells. However, the type of the host cells is not particularly limited, as long as they are capable of replicating recombinant DNA and are capable of expressing the gene of the present enzyme. In the case of utilizing a T7 promoter, the used *Escherichia coli* may be, for example, the *Escherichia coli* BL21(DE3)pLysS. In the case of not using a T7 promoter, the used *Escherichia coli* may be, for example, the *Escherichia coli* JM109. In addition, examples of the yeasts may include the budding yeast SHY2, the budding yeast AH22, the budding yeast INVSc1, and *Pichia pastoris* KM-71H (Invitrogen).

When the present enzyme is prepared, a microorganism containing the recombinant DNA (i.e. a transformant) may be used. The microorganism can be obtained by transfection or transformation using the above-described vector. Such transfection or transformation can be carried out, for example, by a calcium chloride method (Journal of Molecular Biology (J. Mol. Biol.), Vol. 53, p. 159 (1970)), a Hanahan method (J. Mol. Biol., Vol. 166, p. 557 (1983)), an SEM method (Gene, Vol. 96, p. 23 (1990)), a method of Chung, et al. (Proceedings of the National Academy of Sciences of the USA. Vol. 86, p. 2172 (1989)), a calcium phosphate coprecipitation method, electroporation (Potter, H. et al., Proc. Natl. Acad. Sci. U.S.A. 81, 7161-7165 (1984)), and lipofectin (Felgner, P. L. et al., Proc. Natl. Acad. Sci. U.S.A. 84, 7413-7417 (1984)). The above-described microorganism can be utilized to produce the present enzyme.

### 3. Intended uses of the present enzyme agent

A seventh aspect of the present invention relates to an intended use of the present enzyme agent. The present enzyme agent is preferably used for modifying the taste of food products, and in the seventh aspect of the present invention, there is provided a method for producing a food product or a food product material, which includes allowing the present enzyme agent to act on a protein-containing raw material. Also, in the seventh aspect of the present invention, there is provided a method for producing a seasoning, which includes allowing the present enzyme agent to act on at least one type selected from proteins and peptides.

In the method for producing a food product or a food product material of the present invention, the present enzyme agent is allowed to act on a protein-containing raw material. For example, the present enzyme agent is added to a solution containing a protein, and the solution is reacted for a predetermined period of time (for example, 1 hour to 12 hours) under conditions of, for example, 30°C to 70°C, and preferably 40°C to 60°C. As a result of a decomposition reaction by aminopeptidase, which is the active ingredient of the present enzyme agent, specific amino acids (alanine, glycine, etc.) are generated. Although the composition, ratio, etc. of free amino acids in a product can be fluctuated depending on the type, origin, etc. of the substrate used, according to the production method of the present invention, in particular, a food or a food product material that contains relatively large amounts of alanine and glycine is obtained.

In the method for producing a seasoning of the present invention, the present enzyme agent is allowed to act on at least one type selected from proteins and peptides. For example, the present enzyme agent is added to a solution containing at least one type selected from proteins and peptides, and the solution is reacted for a predetermined period of time (for example, 1 hour to 12 hours) under conditions of, for example, 30°C to 70°C, and preferably 40°C to 60°C. As a result of a decomposition reaction by aminopeptidase, which is the active ingredient of the present enzyme agent, specific amino acids (alanine, glycine, etc.) are generated. Although the composition, ratio, etc. of free amino acids in a product can be fluctuated depending on the type, origin, etc. of the substrate used, according to the production method of the present invention, in particular, a seasoning that contains relatively large amounts of alanine and glycine is obtained.

Furthermore, the seventh aspect of the present invention relates to a food product or a seasoning produced by the above-mentioned production method. The food product produced by the above-mentioned production method contains the present enzyme agent and at least one type selected from proteins and peptides. It is to be noted that the enzymes contained in the food product may be inactivated. The food product preferably contains amino acids released from at least one type selected from proteins and peptides. Similarly, the seasoning produced by the above-mentioned production method contains the present enzyme agent and at least one type selected from proteins and peptides. It is to be noted that the enzymes contained in the seasoning may be inactivated. The seasoning preferably contains amino acids released from at least one type selected from proteins and peptides.

The origin of the protein-containing raw material used is not particularly limited, and examples of the origin of the protein-containing raw material may include raw materials derived from: animals (milk, meat, fish, etc.), plants (cereals such as barley, rice, wheat, rye, oats, buckwheat, sorghum, barnyard millet, millet, teff, quinoa, and corn; pulses such as soybeans, lentils, broad beans, peas, chickpeas, mung beans, lupin beans, and kidney beans; and nuts, etc. such as hemp, canary seeds, flaxseed, almonds, cashew nuts, hazelnuts, pecan nuts, macadamia nuts, pistachios, walnuts, Brazil nuts, peanuts, coconuts, pili nuts, chestnuts, sesame seeds, and pine nuts), insects, and microorganisms. Moreover, the origins of the proteins and peptides used are not particularly limited, either, and proteins or peptides derived from the above-mentioned raw materials may be used, or peptides obtained by synthesis or the like may also be used.

After completion of the enzyme reaction with the present enzyme agent, a purification treatment (e.g., filtration, ion exchange, or an activated carbon treatment) may be carried out, as necessary, for the removal of insoluble components, the improvement of purity, decolorization, deodorization, etc.

An eighth aspect of the present invention relates to a method for modifying the taste of a food product, which includes allowing the present enzyme agent to act on a protein-containing raw material. By allowing the present enzyme agent to act on a protein-containing raw material, a decomposition reaction occurs with the aminopeptidase, the active ingredient of the present enzyme agent, and as a result, specific amino acids (alanine, glycine, etc.) are generated. The taste of a food product can be modified by the thus produced amino acids. It is to be noted that the "food product" in the present description includes beverages as well as food products.

### Examples

### < Example 1 >

### 1. Searching for aminopeptidase

*Aspergillus nidulans* A26 strain was subjected to a liquid culture using tripeptone as a sole carbon and nitrogen source. After completion of the culture, proteins secreted into the medium were subjected to SDS-PAGE. The detected extracellular proteins were comprehensively analyzed (secretome), and proteins (HP) with unknown functions were identified, along with known peptidases, by MALDI-TOF/MS.

mRNA was extracted from *Aspergillus nidulans* using RNeasy Plant Mini Kit (QIAGEN), and cDNA (SEQ ID No: 3) was then prepared using PrimeScript^{™} II 1st strand cDNA Synthesis Kit (Takara Bio, Inc.). Based on the published genome information, the cDNA (SEQ ID No: 3) encoding HP was amplified by PCR, and was then fused with pPICZα-A (Invitrogen). The obtained vector was introduced into *Pichia pastoris* KM71H (Invitrogen). Transformants were selected by selecting Zeocin-resistant strains. The transformants were cultured and were then allowed to secrete the target recombinant protein, and the secreted protein was then concentrated and desalted to obtain an HP sample.

The HP sample was allowed to act on alanine (Ala)-pNA (PEPTIDE INSTITUTE, INC.) used as a substrate, and as a result, a decomposition reaction was confirmed. Since the HP sample did not act on the substrate of carboxypeptidase, HP was found to be an aminopeptidase.

### (Method of measuring enzyme activity)

A reaction solution (a 50 mM buffer solution (tris-HCl) pH 8.0, a 1.0 mM substrate (Ala-pNA), and a 0.014 mg/mL enzyme) was reacted at 45°C for 25 minutes. After the enzyme reaction, the amount of released pNA was calculated by measuring the absorbance at a wavelength of 405 nm. The amount of an enzyme that increases the absorbance at a wavelength of 405 nm by 27.5 (path length: 5.56 mm) per minute was defined as 1 Unit (U) (the amount of an enzyme that produces 1 µmol of pNA per minute was defined as 1 U).

### 2. Enzymatic chemical properties of aminopeptidase

### (1) Substrate specificity

The substrate specificity of the present enzyme was examined. Using, as substrates, various types of amino acid-pNA (Ala-pNA, Gly-pNA, Lys-pNA, Val-pNA, Arg-pNA, Phe-pNA, Pro-pNA, Ile-pNA, Glu-pNA, Leu-pNA, and Asp-pNA), the activities thereof were measured. Substrate specificity was evaluated based on the relative activity when the activity value of Ala-pNA, which showed the highest activity, was set as 100%.

The results are shown in Table 1. As shown in Table 1, the aminopeptidase contained in the HP sample had the highest reactivity to alanine. Moreover, it was revealed that the aminopeptidase contained in the HP sample exhibited high reactivity with glycine, lysine, valine, arginine and phenylalanine, in addition to alanine.

**[Table 1]**

| Substrate | Relative activity (%) |
|---|---|
| Ala-pNA | 100 |
| Gly-pNA | 58 |
| Lys-pNA | 45 |
| Val-pNA | 35 |
| Arg-pNA | 33 |
| Phe-pNA | 15 |
| Pro-pNA | 7 |
| Ile-pNA | 7 |
| Glu-pNA | 6 |
| Leu-pNA | 4 |
| Asp-pNA | 3 |

### (2) Optimal temperature

The optimal temperature of the aminopeptidase obtained above was examined. Ala-pNA was used as a substrate, and the activity was measured by changing the temperature applied upon the reaction from 10°C to 80°C. The activity at the temperature condition showing the maximum activity was set as 100%, and the relative amount of activity at each temperature condition was calculated as a relative activity (%).

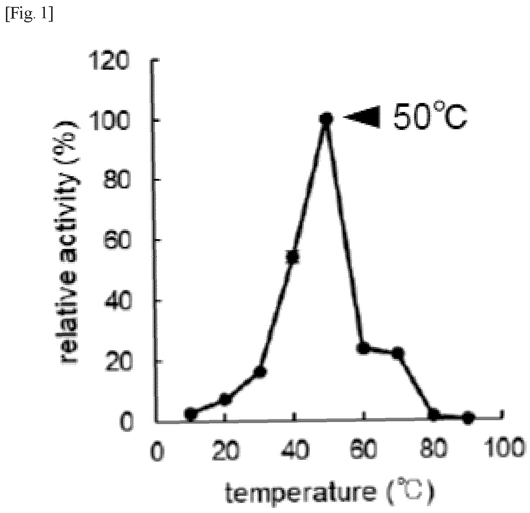

The results are shown in Fig. 1. As shown in Fig. 1, the optimal temperature of the aminopeptidase was 50°C.

### (3) Optimal pH

The optimal pH of the present enzyme was examined. Ala-pNA was used as a substrate, and the pH of the reaction solution was adjusted to be pH 3 to 11, and the activity at each pH was measured. As buffer solutions, a 50 mM McIlvaine buffer solution was used for pH 3 to 7, a 50 mM Tris-HCl buffer solution was used for pH 7 to 8, and a 50 mM CAPS buffer solution was used for pH 8. To 11. The relative amount of activity at each pH condition was calculated as a relative activity (%), when the activity at the pH condition showing the maximum activity was set as 100%.

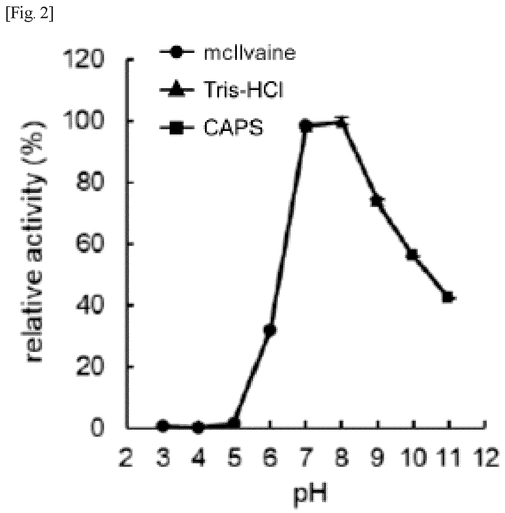

The results are shown in Fig. 2. As shown in Fig. 2, the optimal pH of the aminopeptidase was pH 7 to 8.

### (4) Peptide-decomposing activity

The peptide-decomposing activity of the present enzyme was examined. Using, as substrates, Ala-Ala peptide, Ala-Ala-Ala peptide, Ala-Ala-Ala-Ala-Ala peptide (SEQ ID No: 6), and Lys-Arg-Pro-Pro-Gly-Phe-Ser-Pro-Phe-Arg peptide (SEQ ID No: 7), the aminopeptidase was allowed to act on these substrates. The reaction products were analyzed by TCL or MALDI-TOF-MS.

As a result, generation of Ala was confirmed when any of Ala-Ala peptide, Ala-Ala-Ala peptide and Ala-Ala-Ala-Ala-Ala peptide was used as a substrate. In addition, when Lys-Arg-Pro-Pro-Gly-Phe-Ser-Pro-Phe-Arg peptide was used as a substrate, the cleavage between Lys and Arg at the N-terminus, and the cleavage between Arg and Pro at the N-terminus after Lys release could be confirmed. From the above results, it was confirmed that the present enzyme has aminopeptidase activity also on actual peptides.

In addition, using, as substrates, BAM-12P (Tyr-Gly-Gly-Phe-Met-Arg-Arg-Val-Gly-Arg-Pro-Glu (SEQ ID No: 13; 1424 Da)) and angiotensin III (Arg-Val-Tyr-Ile-His-Pro-Phe (SEQ ID No: 14; 931 Da)), the aminopeptidase was allowed to act on these substrates. The reaction solution used consisted of a 50 mM buffer solution (tris-HCl) pH 8.0, a 0.1 mM substrate (each peptide), 1.0 mM CuSO₄, and a 0.1 µM enzyme, and the reaction was performed at 50°C for 180 minutes. The reaction products were analyzed by TCL or

### MALDI-TOF-MS.

As a result, when BAM-12P (Tyr-Gly-Gly-Phe-Met-Arg-Arg-Val-Gly-Arg-Pro-Glu) was used as a substrate, cleavage was confirmed between Tyr and Gly, between Gly and Gly, and between Gly and Phe at the N-terminus. In addition, when angiotensin III (Arg-Val-Tyr-Ile-His-Pro-Phe) was used as a substrate, cleavage was confirmed between Arg and Val and between Val and Tyr at the N-terminus. From these results as well, it could be confirmed that the present enzyme has aminopeptidase activity also on actual peptides.

### (5) Metal ions

The enzyme activity obtained when various types of metal ions or EDTA used as a metal ion chelator were added to a reaction solution was measured by the following method. The metal ions used were CuCl₂, ZnCl₂, MnCl₂, MgCl₂, CaCl₂, CoCl₂, and FeCl₂.

A substrate solution (a 50 mM buffer solution (tris-HCl) pH 8.0, a 1.0 mM substrate (Ala-pNA), and 1.0 mM various metal ions) was pre-incubated at 45°C for 5 minutes. Thereafter, the enzyme was added to a final concentration of 0.3 mg/mL to the resulting solution, and the reaction was carried out at 45°C for 20 minutes. After addition of the enzyme, the amount of released pNA was calculated by measuring the absorbance at a wavelength of 405 nm.

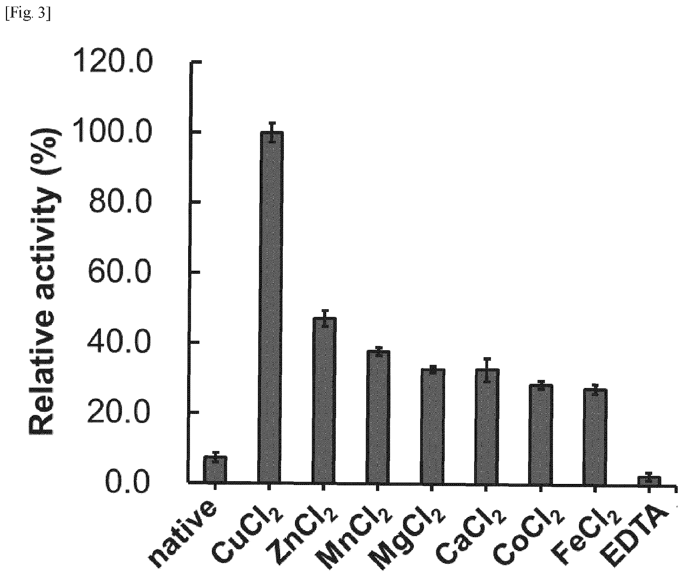

The results are shown in Fig. 3. As shown in Fig. 3, the relative activity was higher in the case of the addition of metal ions than in the case of the non-addition of metal ions (native) or the addition of EDTA. In Fig. 3, the activity value obtained when Ala-pNA was used as a substrate and CuCl₂ was added as metal ions was set as 100%.

### < Example 2 >

### 1. Searching for aminopeptidase

*Aspergillus oryzae* strain was subjected to a liquid culture using tripeptone as a sole carbon and nitrogen source. After completion of the culture, proteins secreted into the medium were subjected to SDS-PAGE. The detected extracellular proteins were comprehensively analyzed (secretome), and proteins (HP) with unknown functions were identified, along with known peptidases, by MALDI-TOF/MS.

mRNA was extracted from *Aspergillus oryzae* using RNeasy Plant Mini Kit (QIAGEN), and cDNA (SEQ ID No: 10) was then prepared using PrimeScript^{™} II 1st strand cDNA Synthesis Kit (Takara Bio, Inc.). Based on the published genome information, the cDNA (SEQ ID No: 10) encoding HP was amplified by PCR, and was then fused with pPICZα-A (Invitrogen). The obtained vector was introduced into *Pichia pastoris* KM71H (Invitrogen). Transformants were selected by selecting Zeocin-resistant strains. The transformants were cultured and were then allowed to secrete the target recombinant protein, and the secreted protein was then concentrated and desalted to obtain an HP sample.

The HP sample was allowed to act on alanine (Ala)-pNA (PEPTIDE INSTITUTE, INC.) used as a substrate, and as a result, a decomposition reaction was confirmed. Since the HP sample did not act on the substrate of carboxypeptidase, HP was found to be an aminopeptidase.

### 2. Enzymatic chemical properties of aminopeptidase

### (Method of measuring enzyme activity)

A reaction solution (a 50 mM buffer solution (tris-HCl) pH 8.0, a 2.5 mM substrate (various types of amino acid-pNA (Ala-pNA, Gly-pNA, Lys-pNA, Val-pNA, Arg-pNA, Phe-pNA, Pro-pNA, Ile-pNA, Glu-pNA, Leu-pNA, and Asp-pNA)), a 0.014mg/mL enzyme, and 1 mM CuCl₂) was prepared, and was then reacted at 45°C for 60 minutes. After the enzyme reaction, the amount of released pNA was calculated by measuring the absorbance at a wavelength of 405 nm. The amount of an enzyme that increases the absorbance at a wavelength of 405 nm by 27.5 (path length: 5.56 mm) per minute was defined as 1 Unit (U) (the amount of an enzyme that produces 1 µmol of pNA per minute was defined as 1 U).

The results are shown in Table 2. Substrate specificity was evaluated based on the relative activity when the activity value of Lys-pNA, which showed the highest activity, was set as 100%. As shown in Table 2, the aminopeptidase contained in the HP sample had the highest reactivity to lysine. Moreover, it was revealed that the aminopeptidase contained in the HP sample exhibited high reactivity with glycine, alanine, valine, arginine and leucine, in addition to lysine.

**[Table 2]**

| Substrate | relative activity (%) |
|---|---|
| Lys-pNA | 100.0 ± 3.6 |
| Gly-pNA | 53.0 ± 1.4 |
| Ala-pNA | 49.3 ± 0.6 |
| Val-pNA | 21.5 ± 1.2 |
| Arg-pNA | 16.3 ± 0.1 |
| Leu-pNA | 14.5 ± 0.6 |
| Phe-pNA | 10.2 ± 0.4 |
| Ile-pNA | 10.2 ± 1.3 |
| Glu-pNA | 5.1 ± 0.4 |
| Asp-pNA | 4.3 ± 1.3 |
| Pro-pNA | 2.2 ± 0.5 |

### Industrial Applicability

The enzyme agent of the present invention contains a novel aminopeptidase as an active ingredient. The enzyme agent of the present invention is suitably used, for example, in the intended use of modification of the taste of a food product, and thus, the present enzyme agent is industrially highly valuable.

The present invention is not limited to the above-described embodiments and explanations of examples of the invention in any way. The present invention includes various modified aspects in a range in which those skilled in the art can be easily conceived without departing from the scope of claims. The contents of the study papers, published patent publications, patent publications, and the like that are explicitly specified in the present description shall be cited by incorporating the entire contents thereof.
SEQ ID No: 1: the amino acid sequence of the present enzyme (mature body sequence)
SEQ ID No: 2: the amino acid sequence of the present enzyme (full-length sequence)
SEQ ID No: 3: the nucleotide sequence of cDNA of the present enzyme (mature body sequence)
SEQ ID No: 4: the nucleotide sequence of cDNA of the present enzyme (full-length sequence)
SEQ ID No: 5: the nucleotide sequence of the genome of the present enzyme (full-length sequence, including introns)
SEQ ID No: 6: peptide (substrate)
SEQ ID No: 7: peptide (substrate)
SEQ ID No: 8: the amino acid sequence of the present enzyme (mature body sequence)
SEQ ID No: 9: the amino acid sequence of the present enzyme (full-length sequence)
SEQ ID No: 10: the nucleotide sequence of cDNA of the present enzyme (mature body sequence)
SEQ ID No: 11: the nucleotide sequence of cDNA of the present enzyme (full-length sequence)
SEQ ID No: 12: The nucleotide sequence of the genome of the present enzyme (full-length sequence, including introns)
SEQ ID No: 13: peptide (substrate)
SEQ ID No: 14: peptide (substrate)

SEQ ID No: 1 
AETMEYLMSLKQQSRERARSQGLFDINRYPDEGAKKCKNGKAGEYSCENVDLLSFLSHQ
ALGSVTREGNDVWGWTSAEGREFGIVGQTDGVAFVEILEDGSLEYVGRLGSQTEPSTWR
DIKVIGDHAYIGSEAAGHGLQIFDLNKLTTASSSKPTVFSTKKDLTAWYRGFGSSHNIVAH
EETNMIYAVGTARNLSCAGGLWMVDVSDPANPTSPGCVNEDGYVHDAQCVIYKGPDEK
YIGQEICFNFNEDTLTIADVTDKKNPIQISKTPYVGASYTHQGWLVDENDHSYLLLDDELD
 
EMDGTGSAANGHTTTYIFDIKDLSAPKHTGTYQSPVRSIDHNQYVVAGLSYQSNYGSGLR
VVDVSSVFEDPTASSFKEVGSFDVHPEDDAVGGEVEFVGSWSVYPFFASGHILLNSIERGI
YSLKYTGPAAEN
SEQ ID No: 2 
MKLSALSLLTVACVATASSHGAETMEYLMSLKQQSRERARSQGLFDINRYPDEGAKKCK
NGKAGEYSCENVDLLSFLSHQALGSVTREGNDVWGWTSAEGREFGIVGQTDGVAFVEIL
EDGSLEYVGRLGSQTEPSTWRDIKVIGDHAYIGSEAAGHGLQIFDLNKLTTASSSKPTVFS
TKKDLTAWYRGFGSSHNIVAHEETNMIYAVGTARNLSCAGGLWMVDVSDPANPTSPGC
VNEDGYVHDAQCVIYKGPDEKYIGQEICFNFNEDTLTIADVTDKKNPIQISKTPYVGASYT
HQGWLVDENDHSYLLLDDELDEMDGTGSAANGHTTTYIFDIKDLSAPKHTGTYQSPVRS
IDHNQYVVAGLSYQSNYGSGLRVVDVSSVFEDPTASSFKEVGSFDVHPEDDAVGGEVEF
VGSWSVYPFFASGHILLNSIERGIYSLKYTGPAAEN
SEQ ID No: 3

gccgagacaatggagtatctcatgtccctgaagcagcaatctcgagaacgtgcccgttcacagggtctgtttgatatcaaccgctaccccgacga
gggagccaagaagtgcaagaacggcaaggcaggagagtactcctgcgagaacgtcgacctcttgtccttcctcagtcaccaggctttgggtagt
gtcacccgtgagggtaacgacgtctggggctggacttctgctgaaggtcgcgagtttggcattgtcggccagactgacggcgtggcctttgttga
gattctggaggacggtagcctcgaatatgttggccgcctgggttctcaaacggaaccatccacgtggcgcgatatcaaggttatcggtgaccatg
cctacattggctccgaggctgcagggcatgggctgcagattttcgacctcaacaagttgacaactgcgtctagctcaaagccgaccgttttctcca
caaagaaggacttgaccgcgtggtataggggcttcggaagctctcataatatcgttgcgcacgaggagaccaatatgatctatgctgttggaaca
gcccgcaacctcagctgcgccggaggcctgtggatggtcgatgtgtctgacccagccaaccctacctcgccgggttgtgtcaacgaagatggtt
atgtgcatgatgcccaatgcgtcatttacaagggccccgatgagaaatatatcggtcaggagatctgcttcaactttaacgaggacacactcaccat
tgccgacgtaaccgacaagaagaacccaattcagatctcgaagaccccctacgtcggcgccagctacactcaccagggctggctcgttgacga
aaacgaccattcctacctcctcctcgacgacgagctcgacgaaatggacggaacgggctccgccgctaacggccacaccaccacctacatctt
cgacatcaaggatctctctgcacccaagcacactggtacctaccagtcccccgtccgctcaatcgaccacaaccagtatgttgtcgcgggtctctc
ttaccagtccaactacggcagcggtctgcgtgtcgtcgatgtcagctctgtctttgaggaccccacggcatctagcttcaaggaagtcggctctttc
gacgtccaccccgaggatgatgcggttggcggtgaggtggagtttgtcggaagctggagtgtttacccgttctttgcgagcgggcatatcttgctc
aacagtattgagcgcggcatttactctctcaagtataccggcccagccgcggagaactag

atgaagctctcggccctatctctgctcaccgtggcctgtgtggcaacagcgtcaagtcatggcgccgagacaatggagtatctcatgtccctgaag
cagcaatctcgagaacgtgcccgttcacagggtctgtttgatatcaaccgctaccccgacgagggagccaagaagtgcaagaacggcaaggc
aggagagtactcctgcgagaacgtcgacctcttgtccttcctcagtcaccaggctttgggtagtgtcacccgtgagggtaacgacgtctggggct
ggacttctgctgaaggtcgcgagtttggcattgtcggccagactgacggcgtggcctttgttgagattctggaggacggtagcctcgaatatgttg
gccgcctgggttctcaaacggaaccatccacgtggcgcgatatcaaggttatcggtgaccatgcctacattggctccgaggctgcagggcatgg
gctgcagattttcgacctcaacaagttgacaactgcgtctagctcaaagccgaccgttttctccacaaagaaggacttgaccgcgtggtatagggg
cttcggaagctctcataatatcgttgcgcacgaggagaccaatatgatctatgctgttggaacagcccgcaacctcagctgcgccggaggcctgt
ggatggtcgatgtgtctgacccagccaaccctacctcgccgggttgtgtcaacgaagatggttatgtgcatgatgcccaatgcgtcatttacaagg
gccccgatgagaaatatatcggtcaggagatctgcttcaactttaacgaggacacactcaccattgccgacgtaaccgacaagaagaacccaatt
cagatctcgaagaccccctacgtcggcgccagctacactcaccagggctggctcgttgacgaaaacgaccattcctacctcctcctcgacgacg
agctcgacgaaatggacggaacgggctccgccgctaacggccacaccaccacctacatcttcgacatcaaggatctctctgcacccaagcaca
ctggtacctaccagtcccccgtccgctcaatcgaccacaaccagtatgttgtcgcgggtctctcttaccagtccaactacggcagcggtctgcgtg
tcgtcgatgtcagctctgtctttgaggaccccacggcatctagcttcaaggaagtcggctctttcgacgtccaccccgaggatgatgcggttggcg
gtgaggtggagtttgtcggaagctggagtgtttacccgttctttgcgagcgggcatatcttgctcaacagtattgagcgcggcatttactctctcaagt
ataccggcccagccgcggagaactag

SEQ ID No: 4 
SEQ ID No: 5

atgaagctctcggccctatctctgctcaccgtggcctgtgtggcaacagcgtcaagtcatggcgccgagacaatggagtatctcatgtccctgaag

cagcaatctcgagaacgtgcccgttcacagggtctgtttgatatcaaccgctaccccgacgagggagccaagaagtgcaagaacggcaaggc

aggagagtactcctgcgagaacgtcgacctcttgtccttcctcagtcaccaggctttgggtagtgtcacccgtgagggtaacgacgtctggggtg

agcactcaacgtccctcaccaagggctgagctaaccgacgtaggctggacttctgctgaaggtcgcgagtttggcattgtcggccagactgacg

gcgtggcctttgttgagattctggaggacggtagcctcgaatatgttggccgcctgggttctcaaacggaaccatccacgtggcgcgatatcaag

gttatcggtgaccatgcctacattggctccgaggctgcagggcatgggctgcagattttcgacctcaacaaggtatggtagcttcccaatttataac

gagcgtctgctgacagccatagttgacaactgcgtctagctcaaagccgaccgttttctccacaaagaaggacttgaccgcgtggtataggggctt

cggaagctctcataatatcgttgcgcacgaggagaccaatatgatctatgctgttggaacagcccgcaacctcagctgcgccggaggcctgtgg

atggtcgatgtgtctgacccagccaaccctacctcgccgggttgtgtcaacgaagatggttatgtgcatgatggtaggttgagggatctccgcagtt

gagaacacgagctgacctactcagcccaatgcgtcatttacaagggccccgatgagaaatatatcggtcaggagatctgcttcaactttaacgag

gacacactcaccattgccgacgtaaccgacaagaagaacccaattcagatctcgaagaccccctacgtcggcgccagctacactcaccagggc

tggctcgttgacgaaaacgaccattcctacctcctcctcgacgacgagctcgacgaaatggacggaacgggctccgccgctaacggccacacc
 
accacctacatcttcgacatcaaggatctctctgcacccaagcacactggtacctaccagtcccccgtccgctcaatcgaccacaaccagtatgtt
gtcgcgggtctctcttaccagtccaactacggcagcggtctgcgtgtcgtcgatgtcagctctgtctttgaggaccccacggcatctagcttcaagg
aagtcggctctttcgacgtccaccccgaggatgatgcggttggcggtgaggtggagtttgtcggaagctggagtgtttacccgttctttgcgagcg
ggcatatcttgctcaacagtattgagcgcggcatttactctctcaagtataccggcccagccgcggagaactag
SEQ ID No: 6
   AAAAA
SEQ ID No: 7
   KRPPGFSPFR
SEQ ID No: 8 
APIMSHLMSIKTEHRERARAQGLFKPNSYIDLAKTPCVDGKAGEYSCENVDLLGFLSHQA
MGSTTREGNDIWGWTSADGREFGIVGQTDGTAFVEVLDDGSLQYVGRLPTQTTATIWRD
MKVIGDHAYIGSESPGHGLQIFDLKKLLETDSNNPTNFSTTEDLTAWYSGFGSSHNIVAHEE
TNMIFAVGTARNLSCAGGLWMIDVSDPANPTSPGCVSEDGYVHDAQCVIYTGPDKEYTN
REICFNYNEDTLTIVDITDRASPIQISKTPYVGASYTHQGWIAVSDMSYLLLDDELDEQDGT
GEAANGHTTTYIFDIKDLANPKHTGTYQSPVRSIDHNQYVIDGLTYQANYGSGLRIVDVSS
VKDDPTGKGFKQVGFFDCHPEDDAQGGEVEFVGAWSVYPYFRSGNILLNSIERGVYSLK
YTGKA
SEQ ID No: 9 
MKPSFVSLLSLACLGAASREGAPIMSHLMSIKTEHRERARAQGLFKPNSYIDLAKTPCVD
GKAGEYSCENVDLLGFLSHQAMGSTTREGNDIWGWTSADGREFGIVGQTDGTAFVEVL
DDGSLQYVGRLPTQTTATIWRDMKVIGDHAYIGSESPGHGLQIFDLKKLLETDSNNPTNFS
TTEDLTAWYSGFGSSHNIVAHEETNMIFAVGTARNLSCAGGLWMIDVSDPANPTSPGCVSE
DGYVHDAQCVIYTGPDKEYTNREICFNYNEDTLTIVDITDRASPIQISKTPYVGASYTHQG
WIAVSDMSYLLLDDELDEQDGTGEAANGHTTTYIFDIKDLANPKHTGTYQSPVRSIDHNQ
YVIDGLTYQANYGSGLRIVDVSSVKDDPTGKGFKQVGFFDCHPEDDAQGGEVEFVGAWS
VYPYFRSGNILLNSIERGVYSLKYTGKA
SEQ ID No: 10 
gctccgattatgagccatttaatgtctattaaaacagaacatcgtgagcgagctcgcgcccagggcttattcaagccaaacagctacattgacctcg
caaagacgccttgcgtggatggcaaagcaggagaatattcctgtgagaacgttgatcttcttggcttcctgagtcatcaggccatgggcagtacga
cccgggaggggaatgatatctggggatggacatcagccgatggccgtgaattcggcatcgttggccaaacggacggaacggcctttgtagaa
gttctggacgatggcagtctgcagtatgtcggccgtctaccgacgcagacaacggcaactatttggagagatatgaaggtgattggggatcatgc
ctatattggatcagagtcaccgggacatggtcttcagatctttgacttgaagaagcttctcgagacggacagcaataacccgaccaatttctcgacc
acggaagacctaacggcttggtacagcggttttggtagctcacacaacatcgttgcgcatgaagagactaacatgatcttcgctgtcggcacagc
caggaacctatcctgcgctggcggcctgtggatgatcgatgtgtctgatcccgcgaacccgacgtcgcctggatgtgtcagtgaagacggttatg
tgcacgatgcacaatgtgtgatctacaccggccccgacaaggaatacacaaaccgtgagatctgcttcaactacaacgaagacaccctcaccat
cgtcgacataacagacagggcctcgccaatccagatctccaaaaccccctatgttggagcaagctacacgcaccaaggctggatagcagtctcc
gacatgtcctacctcctcctggacgacgaattggacgaacaagacgggacaggcgaagccgctaacggacacacaacaacgtacattttcgac
attaaggacctagcgaatccgaaacatacagggacgtaccagtctcccgtgcgatcaatcgaccataaccagtatgttatcgatgggttgacatac
caagctaactacgggagcggattgcgcatcgtcgatgttagctctgttaaggatgatccgacaggcaaggggttcaaacaggtgggtttctttgac
tgccacccagaagatgatgcccagggcggggaagttgagtttgtgggtgcttggagtgtctatccatatttccggagcgggaatattttgttgaaca
gtatcgagaggggtgtctattcgttgaagtacaccggaaaggcttga
SEQ ID No: 11 
atgaaaccatcatttgtctcccttttgtccctggcttgcctcggggcagcctcccgcgagggcgctccgattatgagccatttaatgtctattaaaaca

gaacatcgtgagcgagctcgcgcccagggcttattcaagccaaacagctacattgacctcgcaaagacgccttgcgtggatggcaaagcagga

gaatattcctgtgagaacgttgatcttcttggcttcctgagtcatcaggccatgggcagtacgacccgggaggggaatgatatctggggatggaca

tcagccgatggccgtgaattcggcatcgttggccaaacggacggaacggcctttgtagaagttctggacgatggcagtctgcagtatgtcggcc

gtctaccgacgcagacaacggcaactatttggagagatatgaaggtgattggggatcatgcctatattggatcagagtcaccgggacatggtcttc

agatctttgacttgaagaagcttctcgagacggacagcaataacccgaccaatttctcgaccacggaagacctaacggcttggtacagcggttttg

gtagctcacacaacatcgttgcgcatgaagagactaacatgatcttcgctgtcggcacagccaggaacctatcctgcgctggcggcctgtggatg

atcgatgtgtctgatcccgcgaacccgacgtcgcctggatgtgtcagtgaagacggttatgtgcacgatgcacaatgtgtgatctacaccggccc

cgacaaggaatacacaaaccgtgagatctgcttcaactacaacgaagacaccctcaccatcgtcgacataacagacagggcctcgccaatcca

gatctccaaaaccccctatgttggagcaagctacacgcaccaaggctggatagcagtctccgacatgtcctacctcctcctggacgacgaattgg

acgaacaagacgggacaggcgaagccgctaacggacacacaacaacgtacattttcgacattaaggacctagcgaatccgaaacatacaggg

acgtaccagtctcccgtgcgatcaatcgaccataaccagtatgttatcgatgggttgacataccaagctaactacgggagcggattgcgcatcgtc
 
gatgttagctctgttaaggatgatccgacaggcaaggggttcaaacaggtgggtttctttgactgccacccagaagatgatgcccagggcgggg
aagttgagtttgtgggtgcttggagtgtctatccatatttccggagcgggaatattttgttgaacagtatcgagaggggtgtctattcgttgaagtacac
cggaaaggcttga
SEQ ID No: 12 
atgaaaccatcatttgtctcccttttgtccctggcttgcctcggggcagcctcccgcgagggcgctccgattatgagccatttaatgtctattaaaaca
gaacatcgtgagcgagctcgcgcccagggcttattcaagccaaacagctacattgacctcgcaaagacgccttgcgtggatggcaaagcagga
gaatattcctgtgagaacgttgatcttcttggcttcctgagtcatcaggccatgggcagtacgacccgggaggggaatgatatctggggtatgttaa
ccatactatgcatctgttccgtggacataagatttaacatgaacaggatggacatcagccgatggccgtgaattcggcatcgttggccaaacggac
ggaacggcctttgtagaagttctggacgatggcagtctgcagtatgtcggccgtctaccgacgcagacaacggcaactatttggagagatatgaa
ggtgattggggatcatgcctatattggatcagagtcaccgggacatggtcttcagatctttgacttgaagaaggtatattatcgtaacaatattgtgctc
aatacttgagtacgcccaaggctaa
cggagtccagcttctcgagacggacagcaataacccgaccaatttctcgaccacggaagacctaacggcttggtacagcggttttggtagctcac
acaacatcgttgcgcatgaagagactaacatgatcttcgctgtcggcacagccaggaacctatcctgcgctggcggcctgtggatgatcgatgtg
tctgatcccgcgaacccgacgtcgcctggatgtgtcagtgaagacggttatgtgcacgatggtaggtctctatggttcaatgggtcagctcacagt
ccaatactaacgcaaaccagcacaatgtgtgatctacaccggccccgacaaggaatacacaaaccgtgagatctgcttcaactacaacgaagac
accctcaccatcgtcgacataacagacagggcctcgccaatccagatctccaaaaccccctatgttggagcaagctacacgcaccaaggctgg
atagcagtctccgacatgtcctacctcctcctggacgacgaattggacgaacaagacgggacaggcgaagccgctaacggacacacaacaac
gtacattttcgacattaaggacctagcgaatccgaaacatacagggacgtaccagtctcccgtgcgatcaatcgaccataaccagtatgttatcgat
gggttgacataccaagctaactacgggagcggattgcgcatcgtcgatgttagctctgttaaggatgatccgacaggcaaggggttcaaacaggt
gggtttctttgactgccacccagaagatgatgcccagggcggggaagttgagtttgtgggtgcttggagtgtctatccatatttccggagcgggaa
tattttgttgaacagtatcgagaggggtgtctattcgttgaagtacaccggaaaggcttga
SEQ ID No: 13
   YGGFMRRVGRPE
SEQ ID No: 14
   RVYIHPF

## Claims

1. An enzyme agent containing, as an active ingredient, an aminopeptidase consisting of the amino acid sequence as set forth in SEQ ID No: 1 or an amino acid sequence equivalent thereto.

2. The enzyme agent according to claim 1, in which the aminopeptidase consisting of the equivalent amino acid sequence has an identity of 90% or more to the amino acid sequence as set forth in SEQ ID No: 1 and has an aminopeptidase activity.

3. The enzyme agent according to claim 1, in which the aminopeptidase is derived from *Aspergillus nidulans.*

4. An enzyme agent containing, as an active ingredient, an aminopeptidase, which is derived from *Aspergillus nidulans* and has the following physicochemical properties:
(1) optimal temperature: around 50°C;
(2) optimal pH: around pH 7 to 8; and
(3) substrate specificity: having the highest specificity to L-alanine residue.

5. An enzyme agent containing, as an active ingredient, an aminopeptidase consisting of the amino acid sequence as set forth in SEQ ID No: 8 or an amino acid sequence equivalent thereto.

6. The enzyme agent according to claim 5, in which the aminopeptidase consisting of the equivalent amino acid sequence has an identity of 90% or more to the amino acid sequence as set forth in SEQ ID No: 8 and has an aminopeptidase activity.

7. The enzyme agent according to claim 5, in which the aminopeptidase is derived from *Aspergillus oryzae.*

8. The enzyme agent according to claim 5, which has the highest specificity to L-lysine residue.

9. The enzyme agent according to any one of claims 1 to 8, which is for use in modification of the taste of a food product.

10. A method for producing a food product or a food product material, which includes allowing the enzyme agent according to any one of claims 1 to 8 to act on a protein-containing raw material.

11. A method for producing a seasoning, which includes allowing the enzyme agent according to any one of claims 1 to 8 to act on at least one type selected from proteins and peptides.

12. A method for modifying the taste of a food product, which includes allowing the enzyme agent according to any one of claims 1 to 8 to act on a protein-containing raw material.

13. A food product, containing the enzyme agent according to any one of claims 1 to 8 and at least one type selected from proteins and peptides.

14. A seasoning, containing the enzyme agent according to any one of claims 1 to 8 and at least one type selected from proteins and peptides.
